# EUROPEAN PATENT APPLICATION

(11) **EP 0 839 799 A1**
(43) Date of publication of application: **06.05.1998**
(21) Application number: 96913739.7
(22) Date of filing: 14.05.1996
(51) Int. Cl.: C07C 229/36, C07C 237/20, C07C 309/24, C07C 311/35, C07C 317/28, A61K 31/195, A61K 31/165, A61K 31/215

(54) **2-HYDROXYPHENYLALKYLAMINE DERIVATIVES AND MAILLARD REACTION INHIBITORS**

(30) Priority: 19.05.1995 JP 156620/95
(71) Applicant: KISSEI PHARMACEUTICAL CO., LTD., Matsumoto-City Nagano-Pref. 399 (JP)
(72) Inventor: SATOH, Fumiyasu, Nagano 390 (JP); HARADA, Hiromu 8054, Oaza Nakagawa, Nagano 399-74 (JP); KUSAMA, Hiroshi, Nagano 399 (JP); IYOBE, Akira Medio-Hotaka A101, Nagano 399-83 (JP); KOIZUMI, Takashi Rezidensu-Chino F-202, Minamiazumi-gun Nagano 399-82 (JP); KAMATA, Koji Fureguransu-Merodi A101, Nagano 390 (JP); KATSUNO, Kenji, Nagano 399-06 (JP); KOBAYASHI, Miho 2583, Oaza Minamihotaka, Nagano 399-82 (JP)
(74) Representative: Baverstock, Michael George Douglas
(86) International application number: JP9601261
(87) International publication number: WO9636591

(57) **Abstract**

The present invention relates to a 2-hydroxyphenyl alkylamine derivative represented by the general formula: 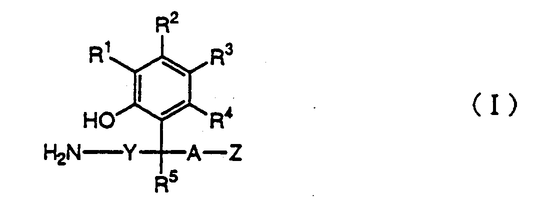 wherein R¹, R², R³ and R⁴ are the same or different: and each represents a hydrogen atom, a lower alkyl group, a lower alkoxy group, a hydroxy group, a mercapto group, a halogen atom, a nitro group, an amino group, an acylamino group, an acyl group or a hydroxy(lower alkyl) group; R⁵ represents a hydrogen atom or a lower alkyl group; A represents a single bond, a lower alkylene group which may have a hydroxy group as a substituent or a lower alkenylene group; Y represents a single bond or a lower alkylene group; and Z represents a carboxyl group, a lower alkoxycarbonyl group, an aryloxycarbonyl group, an aralkyloxycarbonyl group, a carbamoyl group etc. and a pharmaceutically acceptable salt thereof which have a Maillard reaction inhibiting activity.

## Description

The present invention relates to 2-hydroxyphenylalkylamine derivatives which have a Maillard reaction inhibiting activity and are useful as medicaments.

In the field of food chemicals, it is observed in food that reducing sugars such as glucose react with amino compounds to form brown pigments. On the other hand, in recent years it has been found that similar reactions occur *in vivo*. Therefore, a spotlight of attention has been focused on this reaction because it is considered that it plays a major part as one of the factors causing complications of, for example, diabetes and arteriosclerosis.

The above reaction is called the Maillard reaction. The *in vivo* Maillard reaction is considered to progress by a series of reactions which comprise an early stage involving the formation of Schiff bases by non-enzymatical reaction of carbonyl compounds such as reducing sugars, for example, glucose, fructose and pentose, their phosphates and ascorbic acid with free amino groups on body proteins and the subsequent rearrangement into Amadori rearrangement products and advanced stage involving denaturation of the proteins with intermolecular and intramolecular cross-linkage by reactions such as oxidation, dehydration, polymerization and cleavage and the formation of Advanced Glycation End Products (AGE), which are brown and are hardly soluble and which are difficult to decompose by proteases.

The content of AGE and their precursors formed via the above Maillard reaction increases in proportion to the concentration of sugars and proteins and their reaction time. Therefore, it is known that body proteins in patients such as diabetics in whom hyperglycemic conditions exist, long-lived body proteins where the period exposed to sugars is long, body proteins where the speed of metabolizing is low and proteins in blood and tissue in patients with renal diseases where clearance is slow are susceptible to the Maillard reaction.

Accordingly, as examples of susceptible body proteins, lens crystallin of eyeballs, serum albumin, collagen and elastin in connective tissues such as skin and vascular walls, nervous myelin, hemoglobin and glomerular basement membrane in kidneys are illustrative. The Maillard reaction is considered as one of the factors causing complications in diabetes and diseases resulting from aging, for example, retinopathy, nephropathy, neuropathy, cardiovascular disorders, cataract and arteriosclerosis, which cause denaturation, hypergasia or functional disorders of these proteins. Furthermore, recently, it has been reported that the Maillard reaction is also a factor causing dialytic amyloidosis, which occurs frequently in patients receiving long dialysis and having Alzheimer's disease. Thus, research has been directed towards finding compounds having a Maillard reaction inhibiting activity for the prevention and treatment of these diseases.

The present invention relates to a 2-hydroxyphenylalkylamine derivative represented by the general formula: 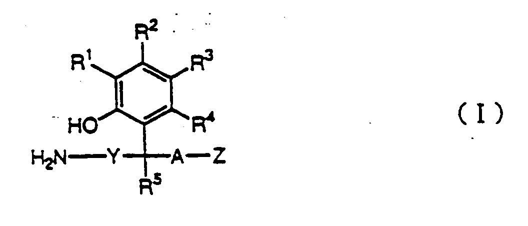 wherein R¹, R², R³ and R⁴ are the same or different and each represents a hydrogen atom, a lower alkyl group, a lower alkoxy group, a hydroxy group, a mercapto group, a halogen atom, a nitro group, an amino group, an acylamino group, an acyl group or a hydroxy(lower alkyl) group; R represents a hydrogen atom or a lower alkyl group; A represents a single bond, a lower alkylene group which may have a hydroxy group as a substituent or a lower alkenylene group; Y represents a single bond or a lower alkylene group; and Z represents a carboxyl group, a lower alkoxycarbonyl group, an aryloxycarbonyl group, an aralkyloxycarbonyl group, a carbamoyl group, a mono or di(lower alkyl)aminocarbonyl group, a mono or diarylaminocarbonyl group, a mono or diaralkyl aminocarbonyl group, a cyano group, a sulfo group, a lower alkylsulfinyl group, a lower alkylsulfonyl group, an aryl sulfinyl group, an arylsulfonyl group, an aralkylsulfinyl group, an aralkylsulfonyl group, a sulfamoyl group, a lower alkylsulfamoyl group, an arylsulfamoyl group or an aralkylsulfamoyl group and a pharmaceutically acceptable salt thereof.

The present invention also relates to a pharmaceutical composition comprising the above 2-hydroxyphenylalkylamine derivative or pharmaceutically acceptable salt thereof.

The present invention additionally relates to a Maillard reaction inhibitor comprising, as an active ingredient, the above 2-hydroxyphenylalkylamine derivative or pharmaceutically acceptable salt thereof.

The present invention relates as well to a use of the above 2-hydroxyphenylalkylamine derivative or pharmaceutically acceptable salt thereof for the manufacture of a pharmaceutical composition for the prevention or treatment of diseases caused by Maillard reaction.

Furthermore, the present invention relates to a use of the above 2-hydroxyphenylalkylamine derivative or pharmaceutically acceptable salt thereof as a Maillard reaction inhibitor.

In the above 2-hydroxyphenylalkylamine derivatives of the present invention, the term "lower alkyl group" means a straight- or branched-chain alkyl group having from 1 to 6 carbon atoms such as a methyl group, an ethyl group, a propyl group, an isopropyl group, a butyl group, an isobutyl group, asec-butyll group, a tert-butyl group, a pentyl group, an isopentyl group, a neopentyl group, a tert-pentyl group and a hexyl group, and the term "lower alkoxy group" means a straight- or branched-chain alkoxy group having from 1 to 6 carbon atoms such as amethoxy group,anethoxy group, a propoxy group, an isopropoxy group, a butoxy group, an isobutoxy group, a sec-butoxy group, a tert-butoxy group, a pentyloxy group, an isopentyloxy group, a neopentyloxy group, a tert-pentyloxy group and a hexyloxy group.

The term "aryl group" means an aromatic hydrocarbon group such as a phenyl group and a naphthyl group, and the term "aralkyl group" means the above lower alkyl group substituted by the above aryl group.

The term "halogen atom" means a fluorine atom, a chlorine atom, a bromine atom and an iodine atom, and the term "acyl group" means an alkylcarbonyl group having from 2 to 7 carbon atoms and having a straight-or branched-chain alkyl group such as an acetyl group, a propionyl group and a butyryl group.

The term "lower alkylene group" means a straight- or branched-chain alkylene group having from 1 to 6 carbon atoms such as a methylene group, an ethylene group, a propylene group, a trimethylene group, a tetramethylene group, a pentamethylene group and a hexamethylene group, and the term "lower alkenylene group means a straight- or branched-chain alkenylene group having from 2 to 6 carbon atoms such as a vinylene group and a propenylene group.

Some of the 2-hydroxyphenylalkylamine derivatives represented by the above general formula (I) of the present invention are known compounds and are described in the literature. The 2-hydroxyphenylalkylamine derivatives represented by the above general formula (I) of the present invention can be prepared by methods described in the literature (for example, published Japanese patent applications (kokai) Nos.sho46-7875, sho48-67245, sho52-36644, sho53-135951 and sho56-150040, U.S. Patent No.3,551,476, J.Agric.Food.Chem., Vol.25, No.4, p.965(1977), Org. Prep. Proced., Vol.l, No.4, pp.271-277 (1969), J. Prakt. Chem., Vol.30 (1-2),pp.18-38 (1965), Zhur.ObshcheiRhim.Vol.28, pp.l371-1374 (1958), Chemistry Letters, pp.687-690 (1987), Tetrahedron Letters, Vol.23, No.51, pp.5399-5402 (1982), Tetrahedron Letters, Vol.25, No.41, pp.4583-4586 (1984), Tetrahedron Letters, Vol.32, No.17, pp.1963-1964 (1991), Ann. Chem., Vol.ll, p.l952 (1982)), analogous methods thereto or these methods combined with other known methods.

For example, of the compounds of the present invention, the compounds represented by the general formula: 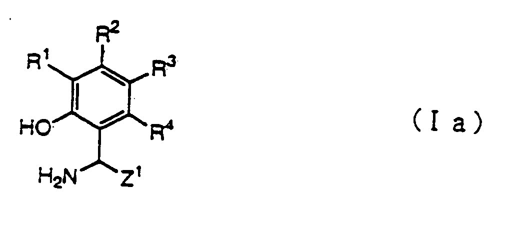 wherein Z¹ represents a carboxyl group, a lower alkoxycarbonyl group, an aryloxycarbonyl group, an aralkyloxycarbonyl group, a carbamoyl group, a mono or di(lower alkyl)aminocarbonyl group, a mono or diarylaminocarbonyl group or a mono or diaralkyl aminocarbonyl group; and R¹, R², R³ and R⁴ have the same meanings as described above can be prepared by allowing a benzaldehyde derivative represented by the general formula: 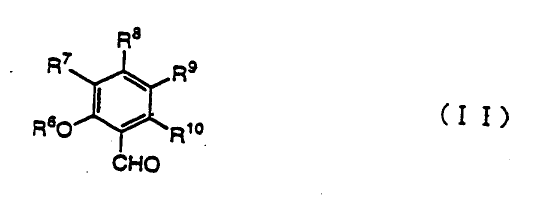 wherein R¹ represents a hydroxy-protective group; and R⁷, R⁸, R⁹ and R¹⁰ are the same or different and each represents hydrogen atom, a lower alkyl group, a lower alkoxy group, a protected hydroxy group, protected mercapto group, a halogen atom, a nitro group, a protected amino group, an acylamino group, an acyl group or a protected hydroxy(lower alkyl) group to react with ammonium carbonate and sodium cyanide in an inert solvent to give a hydantoin derivative represented by the general formula: 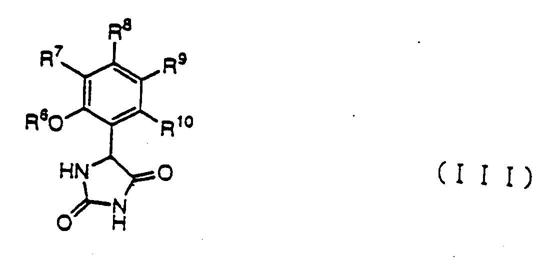 wherein R⁶, R₇, R⁸, R⁹ and R¹⁰ have the same meanings as described above, hydrolyzing under an alkaline condition, protecting the amino group etc. with an appropriate protective group in he usual way as occasion demands, subjecting the resulting compound to esterification using an alcohol compound represented by the general formula:

R¹¹-OH (IV)

wherein R¹¹ represents a lower alkyl group, an aryl group or an aralkyl group in the usual way, protecting the amino group with an appropriate protective group in the usual way as occasion demands, subjecting the resulting compound to amidation using an amine compound represented by the general formula: 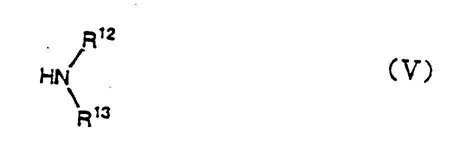 wherein R¹² and R¹³ are the same or different and each represents a hydrogen atom, a lower alkyl group, an aryl group and an aralkyl group in the usual way as occasion demands, and removing the protective group of the hydroxy group, and so on.

Of the compounds of the present invention, a compound represented by the general formula: 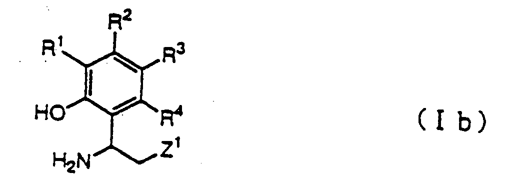 wherein R⁷, R⁸, R⁹, R⁴ and Z¹ have the same meanings as described above can be prepared by allowing a compound represented by the general formula: 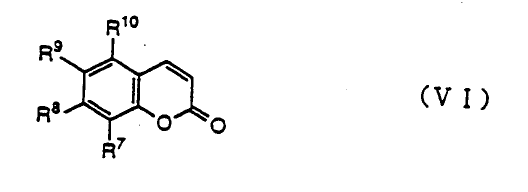 wherein R⁷, R⁸, R⁹ and R¹⁰ have the same meanings as described above to react with ammonia, opening the ring, protecting the amino group or other group with an appropriate protective group in the usual way as occasion demands, subjecting the resulting compound to esterification using the alcohol compound represented by the above general formula (IV) in the usual way, protecting the amino group with an appropriate protective group in the usual way as occasion demands, subjecting the resulting compound to amidation using the amine compound represented by the above general formula (V) in the usual way as occasion demands, and removing the protective groups.

Of the compounds of the present invention, a compound represented by the general formula: 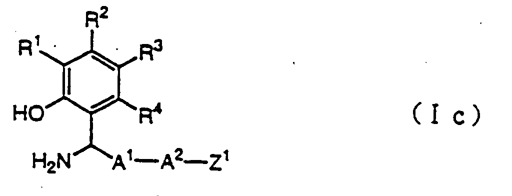 wherein A¹ represents a single bond or an alkylene group having from 1 to 4 carbon atoms; A represents an ethylene group or a vinylene group; and R¹, R², R³, R⁴ and Z¹ have the same meanings as described above can be prepared by reducing an ester compound represented by the general formula: 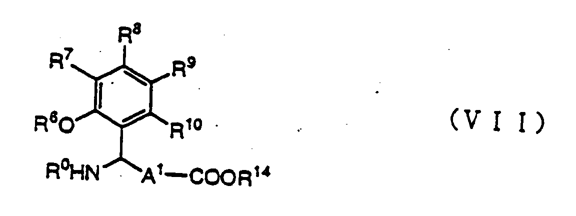 wherein R⁰ represents an amino-protective group; R¹⁴ represents a lower alkyl group; and A¹, R⁶, R⁷, R⁸, R⁹ and R¹⁰ have the same meanings as described above using an appropriate reducing agent such as sodium borohydride or lithium aluminum hydride to give an alcohol compound represented by the general formula: 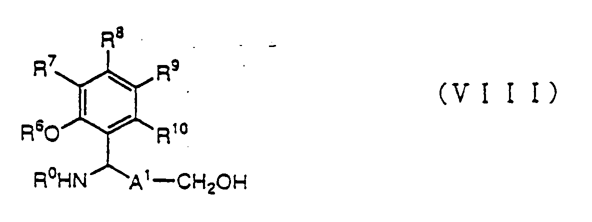 wherein A¹, R⁰, R⁶, R⁷, R⁸, R⁹ and R¹⁰ have the same meanings as described above, oxidizing using an appropriate oxidizing agent such as sulfur trioxide pyridine complex in dimethyl sulfoxide to give an aldehyde compound represented by the general formula: 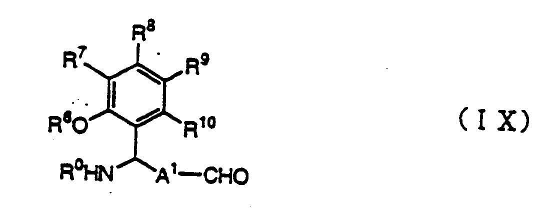 wherein A¹, R⁰, R⁶, R⁷, R⁸, R⁹ and R¹⁰ have the same meanings as described above, allowing to react with alkyl phosphite represented by the general formula: 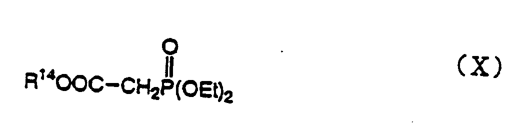 wherein R¹⁴ has the same meaning as described above (Horner-Emons reaction) to give a compound represented by the general formula: 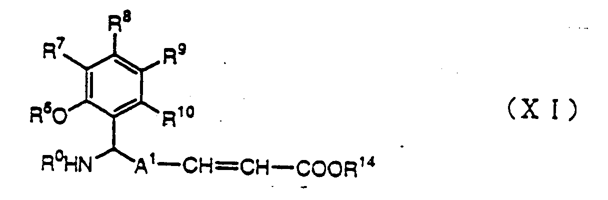 wherein A¹, R⁰, R⁶, R⁷, R⁸, R⁹, R¹⁰ and R¹⁴ have the same meanings as described above, reducing using an appropriate reducing agent such as catalytic hydrogenation with a nickel catalyst or a palladium catalyst to give a compound represented by the general formula: 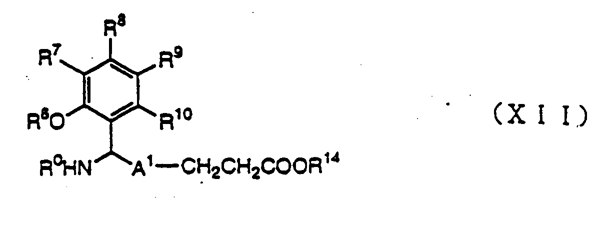 wherein A¹, R⁰, R⁶, R⁷, R⁸, R⁹, R¹⁰ and R¹⁴ have the same meanings as described above, hydrolyzing or subjecting the resulting compound to amidation using the amine compound represented by the above general formula (V) in the usual way as occasion demands, and removing the protective group.

Of the compounds of the present invention, a compound represented by the general formula: 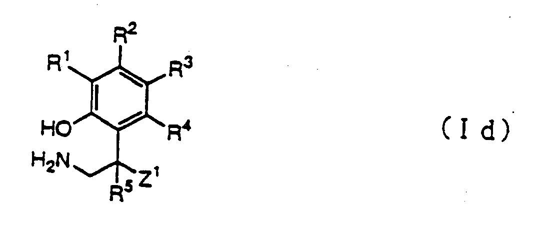 wherein R¹, R², R³, R⁴, R⁵ and Z¹ have the same meanings as described above can be prepared by treating a nitrile compound represented by the general formula: 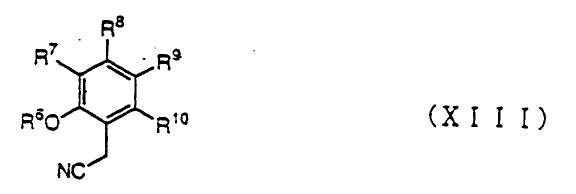 wherein R⁶, R⁷, R⁸, R⁹ and R¹⁰ have the same meanings as described above in the presence of sodium ethoxide in diethyl carbonate and ethanol to give a compound represented by the general formula: 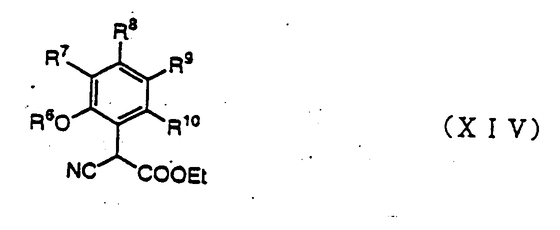 wherein R⁶, R⁷, R⁸, R⁹ and R¹⁰ have the same meanings as described above, allowing to react with an alkylhalide compound represented by the general formula:

R⁵-X (XV)

wherein X represents a halogen atom; and R⁵ has the same meaning as described above as occasion demands, hydrolyzing or subjecting the resulting compound to amidation using the amine compound represented by the general formula (V) in the usual way as occasion demands, reducing using an appropriate reducing agent such as catalytic hydrogenation with a nickel catalyst or a palladium catalyst, protecting the amino group with an appropriate protective group in the usual way as occasion demands, subjecting the resulting compound to esterification or ester interchange using the alcohol compound represented by the above general formula (IV) in the usual way as occasion demands, and removing the protective group. Of the compounds of the present invention, a compound represented by the general formula: 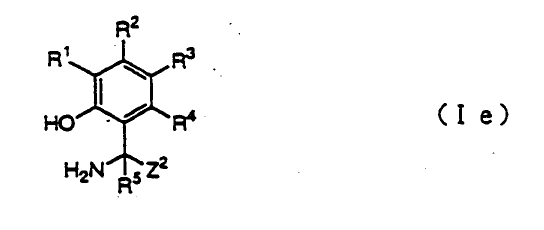 wherein Z² represents a carboxyl group, a lower alkoxycarbonyl group, an aryloxycarbonyl group, an aralkyloxycarbonyl group, a carbamoyl group, a mono or di(lower alkyl)aminocarbonyl group, a mono or diarylaminocarbonyl group, a mono or diaralkylaminocarbonyl group or a cyano group; and R¹, R², R³, R⁴ and R⁵ have the same meanings as described above can be prepared by allowing a compound represented by the general formula: 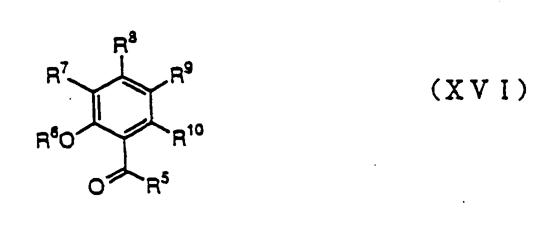 wherein R⁵, R⁶, R⁷, R⁸, R⁹ and R¹⁰ have the same meanings as described above to react with potassium cyanide and ammonium chloride to give a nitrile compound represented by the general formula: 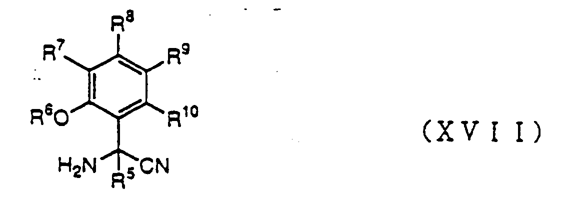 wherein R⁵, R⁶, R⁷, R⁸, R⁹ and R¹⁰ have the same meanings as described above, hydrolyzing in the usual way as occasion demands, protecting the amino group with an appropriate protective group in the usual way as occasion demands, subjecting the resulting compound to esterification using the alcohol compound represented by the above general formula (IV) or to amidation using the amine compound represented by the above general formula (V) in the usual way as occasion demands, and removing the protective group. of the present invention, a compound represented by the general formula:

Of the compounds of the present invention, a compound represented by the general formula: 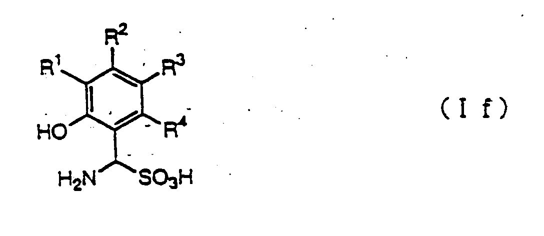 wherein R¹, R², R³ and R⁴ have the same meanings as described above can be prepared by allowing a benzaldehyde compound represented by the above general formula (II) to react with sodium hypophosphite and ammonia or ammonium chloride to give a compound represented by the general formula: 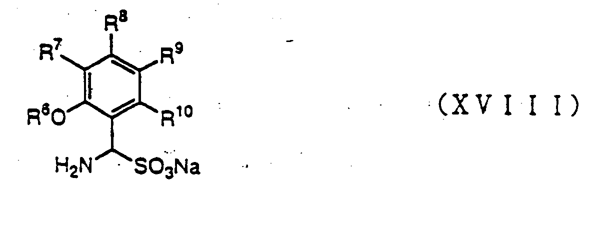 wherein R⁶, R⁷, R⁸, R⁹ and R¹⁰ have the same meanings as described above, and removing the protective group.

Of the compounds represented by the above general formula (I) of the present invention, an optical isomer can be separated from the corresponding racemic compound by chiral column chromatography or by treating suitably according to a diastereomeric resolution procedure.

The compounds of the present invention obtained by the above production processes can be easily isolated and purified by conventional separation means such as fractional recrystallization, purification by column chromatography and solvent extraction.

The 2-hydroxyphenylalkylamine derivatives represented by the above general formula (I) of the present invention can be converted into pharmaceutically acceptable salts thereof in the usual way. Examples of such salts include acid addition salts with mineral acids such as hydrochloric acid, hydrobromic acid, hydroiodic acid, sulfuric acid, nitric acid and phosphoric acid, acid addition salts with organic acids such as formic acid, acetic acid, methanesulfonic acid, benzenesulfonic acid, p-toluenesulfonic acid, propionic acid, citric acid, succinic acid, tartaric acid, fumaric acid, butyric acid, oxalic acid, malonic-acid, maleic-acid, lactic-acid, malic-acid, carbonic-acid, glutamic acid and aspartic acid, salts with inorganic bases such as a sodium salt, a potassium salt and a calcium salt, and salts with organic bases such as morpholine, and piperidine or amino acids.

In addition, the compounds presented by the above general formula (I) of the present invention also include hydrates thereof and solvates thereof with pharmaceutically acceptable solvents such as ethanol.

The 2-hydroxyphenylalkylamine derivatives represented by the above general formula (I) of the present invention have one asymmetric carbon atom at least, and therefore, two isomeric forms of (R) configuration and (S) configuration based on each asymmetric carbon atom exist. Either one of the isomers or a mixture thereof can be employed in the present invention.

Of the compounds represented by the above general formula (I) of the present invention, the compounds having an unsaturated bond exist in two geometrical isomer forms. Either the cis (Z) isomer or trans (E) isomer can be employed in the present invention.

Of the compounds represented by the above general formula (I) of the present invention, compounds wherein A represents a single bond are preferred, and compounds wherein Y represents a single bond are preferred.

Of the compounds represented by the above general formula (I) of the present invention, as examples of preferred compounds, α -amino-2-hydroxyphenylacetic acid, methyl α -amino-2hydroxyphenylacetate, α -amino-5-chloro-2-hydroxyphenylacetic acid, α -amino-2-hydroxy-5-methoxyphenylacetic acid, methyl α -amino-2,5-dihydroxyphenylacetate, N,N-dimethyl- α amino-2-hydroxyphenylacetamide, α -amino-2-hydroxyphenylacetamide, N-benzyl- α -amino-2-hydroxyphenyl-acetamide, N(2-phenethyl)-α -amino-2-hydroxyphenylacetamide, optical isomers thereof and pharmaceutically acceptable salts thereof.

In the *in vitro* Maillard reaction inhibiting activity test using lysozyme and fructose, the compounds represented by the above general formula (I) of the present invention exhibit effects of inhibiting dimerization of lysozyme greater than aminoguanidine, which is known as a Maillard reaction inhibitor.

In the *in vivo* AGE formation in plasma proteins inhibiting activity test using streptozotocin-induced diabetic rats, the compounds of the present invention exhibit effects of inhibiting AGE formation greater than aminoguanidine and exhibit the effect of decreasing the content of formed AGE significantly in comparison with the control group (untreated group).

Thus, the compounds represented by the above general formula (I) of the present invention and pharmaceutically acceptable salts thereof have excellent Maillard reaction inhibiting activities and are very useful compounds as agents for the prevention or treatment of diseases caused by the Maillard reaction.

The compounds represented by the above general formula (I) of the present invention and pharmaceutically acceptable salts thereof have Maillard reaction inhibiting activities and are effective against diseases caused by the Maillard reaction. As examples of such diseases, complications of diabetes such as retinopathy, nephropathy, neuropathy, coronary cardiopathy, peripheral cardiovascular disorders, cerebrovascular disorders, arteriosclerosis, arthrosclerosis, cataract, coagulation disorders and osteopenia, diseases which are considered to result from aging such as atherosclerosis, glomerulonephritis, senile cataract, osteoarthritis, periarticular tetania, arthrosclerosis, senile osteoporosis and Alzheimer's diseases and dialytic amyloidosis are illustrative, and the compounds of the present invention are useful as agents for the prevention or treatment of said diseases. As is commonly known, Maillard reactions take place in cosmetics and foods containing proteins and amino acids, and deterioration of proteins and amino acids occurs. Therefore, the compounds of the present invention are useful as Maillard reaction inhibitors in cosmetics and foods.

In acute toxicity tests using rats, no death was observed by a single administration, for example, at the dose of 2000mg/kg of α -amino-2-hydroxyphenylacetic acid hydrochloride, and a significant effect was not observed in comparison with the control group (untreated group) in hematological examinations on the seventh day after administration.

In addition, in toxicity tests using rats, significant toxicity and side effects were not observed by administration at the dose of 100mg/kg/day of α -amino-2-hydroxyphenylacetic acid hydrochloride for ten consecutive weeks. Thus, the compounds of the present invention are very safe compounds, and therefore, are very useful as Maillard reaction inhibitors.

When the compounds represented by the above general formula (I) of the present invention and pharmaceutically acceptable salts thereof are employed in practical treatment, they are administered orally or parenterally in the form of appropriate pharmaceutical compositions such as tablets, powders, fine granules, granules, capsules, liquids and solutions, injections, external preparations, ophthalmic solutions or suppositories. These pharmaceutical compositions can be formulated in accordance with conventional methods using conventional pharmaceutical carriers, excipients and other additives.

Of the above pharmaceutical compositions, in formulating tablets, powders, fine granules, granules, and capsules, conventional excipients, disintegrators, binders and lubricants can be used. Sugars or sugar alcohols such as D-mannitol, lactose and sucrose, starches or starch derivatives such as wheat starch, rice starch, corn starch, potato starch, α -starch, partial α -starch, dextrin, cyclodextrin, pullulan, and hydroxypropylstarch, celluloses or cellulose derivatives such as crystalline cellulose, crystalline cellulose.carmellose sodium, methylcellulose and hydroxypropylmethylcellulose, sodium alginate, acacia, agar, macrogol, aluminium stearate and aluminium monostearate can be used as excipients, and calcium hydrogenphosphate, anhydrous calcium hydrogenphosphate, magnesium aluminometasilicate, synthetic aluminum silicate, synthetic hydrotalcite, aluminum hydroxide, magnesiumhydroxide, calcium phosphate, dried aluminum hydroxide gel, precipitated calcium carbonate and light anhydrous silicic acid can be used as mineral excipients. The application of these materials are not limited to use as excipients, and these materials can be used as disintegrators or binders.

Carmellose calcium, carmellose, low substituted hydroxypropylcellulose, sodium carboxymethylstarch, croscarmellose sodium, tragacanth, starches or starch derivatives such as wheat starch, rice starch, corn starch, potato starch, α-starch, partial α -starch, dextrin, pullulan, and hydroxypropylstarch can be used as disintegrators. The application of these materials are not limited to use as disintegrators, and these materials can be used as excipients.

Hydroxyethylcellulose, hydroxypropylcellulose, polyvinyl alcohol, polyvinylpyrrolidone, starches or starch derivatives such as wheat starch, rice starch, corn starch, potato starch, α -starch, partial α -starch, dextrin, pullulan and hydroxypropylstarch can be used as binders.

Calcium stearate, magnesium stearate, stearic acid, talc, cetanol, polyoxy 40 stearate, leucine, hydrogenated oil, sodium lauryl sulfate, paraffin and polyoxyethyleneglycol fatty acid ester fatty acid ester can be used as lubricants. The application of these materials are not limited to use as lubricants, and these materials can be used as excipients.

In formulating tablets, the tablets can be coated with lactose, saccharose, gelatin, hydroxypropylcellulose, hydroxyropylmethylcellulose, polyvinylacetal diethylaminoacetate, metaacrylic acid copolymer and hydroxypropylmethylcellulose phthalate.

In formulating liquids and solutions, purified water, poly alcohol, saccharose, invert sugar and dextrose can be used as diluents. These preparations may further contain dissolving aids, moistening agents, suspending agents, sweeteners, flavors, fragrances and antiseptics.

In formulating injections, purified water, isotonic sodium chloride solution, alcohol, glycerin, poly alcohol, and vegetable oil can be used as diluents. These preparations may further contain buffers, isotonicities, antiseptics, moistening agents, emulsifiers, dispersing agents, stabilizing agents, and dissolving aids.

In formulating ophthalmic solutions, these preparations may optionally contain buffers, isotonicities, stabilizing agents, preservatives, antioxidants, thickeners, antiseptics and dissolving aids in addition to diluents.

In formulating suppositories, lipid, wax, semisolid or liquid poly alcohol, natural oil and hydrogenated oil can be used as carriers. These preparations may further contain dispersing agents, auxiliary dispersing agents and absorbing enhancers.

The dosage is appropriately decided depending on the sex, age, body weight and severity of symptoms of each patient to be treated, which is approximately within the range of from 1 to 1,000 mg per day per adult human in the case of oral administration and approximately within the range of from 0.1 to 100 mg per day per adult human in the case of parenteral administration, and the daily dosage can be divided into one to several doses per day.

When the compounds represented by the above general formula (I) of the present invention are employed as ophthalmic solutions, these preparations may be formulated within the range of from O.05 to 5 W/V% in accordance with conventional methods, and frequency of administration is appropriately decided depending on the severity of the symptoms and other factors of each patient to be treated.

When the compounds represented by the above general formula (I) of the present invention are employed as external medicines or cosmetics, these preparations may be formulated approximately within the range of from 0.05 to 10 parts by weight to the total weight using conventional external medicinal bases or cosmetic bases in accordance with conventional methods. Furthermore, the compounds of the present invention can be formulated for use of food in accordance with conventional methods and can be used by adding to food.

### Examples

The present invention is further illustrated in more detail by way of the following Reference Examples, Examples and Test Examples.

### Reference Example 1

### 2-Methoxymethoxybenzaldehyde

N,N-Diisopropylethylamine (23.5ml) and a solution of chloromethyl methyl ether (10.3ml) in dichloromethane (2Oml) were added dropwise to a solution of salicylaldehyde (15g) in dichloromethane (150ml) under ice-cooling, and the mixture was stirred for 2 hours at room temperature. After the reaction, the reaction mixture was washed successively with a 2N aqueous sodium hydroxide solution, brine, a 10% aqueous citric acid solution and brine, dried over magnesium sulfate, and concentrated *in vacuo*. The obtained residue was purified by column chromatography on silica gel to give 2-methoxymethoxybenzaldehyde (20.4g) as a colorless oil.
NMR(CDC1₃,270MHz)
δ ppm : 3.52(3H,s), 5.31(2H,s), 7.00-7.15(1H,m), 7.22(1H,d,J=7.9Hz),7.45-7.60(1H,m),7.85(1H,dd,J =7.4Hz,2.0Hz),10.51(1H,brd,J=l.OHz)

### Reference Example 2

### 5-(2-Methoxymethoxypheny)hydantoin

A solution of 2-methoxymethoxybenzaldehyde (lOg) in ethanol (75ml) was added to a solution of ammonium carbonate (20.2g) and sodium cyanide (4.43g) in water (75ml), and the mixture was stirred at 50 C for 2 days. After the reaction, the reaction mixture was concentrated to about a half volume *in vacuo*. The concentrate was cooled with ice, and the resulting precipitates were collected by filtration, washed successively with water and diethyl ether, and dried *in vacuo* with phosphorus pentoxide to give 5-(2-methoxymethoxyphenyl)hydantoin (7.4g) as a white powder.
NMR(DMSO-d₆,400MHz)
δ ppm : 3.36(3H,s), 5.18(2H,s), 5.20(1H,s), 6.96-7.04(1H,m), 7.09(lH,d,J=8.2Hz), 7.25(lH,dd,J=7.6Hz,1.6Hz), 7.28-7.36(1H,m), 8.06(1H,brs), 10.68(1H,brs)

### Reference Example 3

### α -tert-Butoxycarbonylamino-2-methoxymethoxyphenylacetic acid

5-(2-Methoxymethoxyphenyl)hydantoin (4.0g) was added to an aqueous sodium hydroxide (2.02g) solution (40ml), and the mixture was heated under reflux for 2 days. After the reaction, 2N hydrochloric acid (31.9ml) was added to the reaction mixture under ice-cooling, and the mixture was evaporated *in vacuo* until gas evolution stopped. To the residue were added dioxane(30ml), triethylamine (3.24ml) and di-tert-butyl dicarbonate (4.06g), and the mixture was stirred for 1 day at room temperature. After the reaction, chloroform and a small amount of methanol were added to the reaction mixture, and the mixture was washed successively with a 10% aqueous citric acid solution and brine, dried over magnesium sulfate, and concentrated *in vacuo*. The obtained residue was purified by column chromatography on silica gel to give α tert-butoxycarbonylamino-2-methoxymethoxyphenyl-acetic acid (3.65g) as a colorless amorphous powder.
NMR(CDCl₃,400MHz)
δ ppm : 1.43(9H,s), 3.46(3H,brs), 5.21(1H,d,J=6.7Hz), 5.25 (lH,d,J=6.7Hz), 5.60(lH,br), 5.66(lH,br), 7.02 (lH,t,J=7.5Hz), 7.13(1H,d,J=8.3Hz), 7.24-7.36(2H,m)

### Reference Example 4

### Methyl α -tert-butoxycarbonylamino-2-methoxymethoxyphenylacetate

A solution of diazomethane in diethyl ether was added dropwise to a solution of α -tert-butoxycarbonylamino-2-methoxymethoxyphenylacetic acid (2.0g) in methanol (lOml) under ice-cooling. After the reaction, the reaction mixture was concentrated *in vacuo*, and the obtained residue was purified by column chromatography on silica gel to give methyl α -tert-butoxycarbonylamino-2-methoxymethoxyphenylacetate (1.9lg) as a white solid.
NMR(CDCl₃,400MHz)
δ ppm: 1.43(9H,s),3.46(3H,s),3.69(3H,s),5.18(1H,d,J= 6.7Hz), 5.22(1H,d,J=6.7Hz), 5.53(1H,brd,J=8.9Hz), 5.65 (lH,brd,J=7.9Hz), 7.00(1H,dt,J=7.4Hz,l.OHz), 7.11 (lH,d,J=8.3Hz), 7.23-7.35(2H,m)

### Reference Example 5

### α - tert-Butoxycarbonylamino-2,5-dimethoxymethoxyphenylacetic acid.

In the same manner as that in Reference Examples 1-3, α -tert-butoxycarbonylamino-2,5-dimethoxymethoxyphenylacetic acid was obtained by the use of 2,5-dihydroxybenzaldehyde as a starting material.
a yellow oil
NMR(DMSO-d₆,400MHz)
δ ppm : 1.38(9H,s), 3.36(3H,s), 3.38(3H,s), 5.05-5.19(4H,m), 5.47(lH,d,J=8.8Hz), 6.88-6.95(1H,m), 6.98-7.04(2H,m), 7.32(lH,brd,J=9.OHz), 12.6(lH,br)

### Reference Example 6

### N,N-Dimethyl- α -tert-butoxycarbonylamino-2-methoxymethoxyphenylacetamide

Dimethylamine-hydrochloride (109mg), triethylamine (0.17ml) and diethyl cyanophosphonate (219mg) were added successively to a solution of α -tert-butoxycarbonylamino-2-methoxymethoxyphenylacetic acid (380mg) in acetonitrile (lOml) under ice-cooling, and the mixture was stirred for 1 day being allowed to warm to room temperature. After the reaction, dichloromethane was added to the reaction mixture. The mixture was washed successively with a 10% aqueous citric acid solution, brine, a saturated aqueous sodium hydrogen carbonate solution and brine, dried over magnesium sulfate, and concentrated *in vacuo*. The obtained residue was purified by column chromatography on silica gel to give N,N-dimethyl- α -tert-butoxycarbonylamino-2-methoxymethoxyphenylacetamide (256mg) as a white powder.
NMR(CDC1₃,400MHz)
δ ppm : 1.40(9H,s), 2.92(3H,s), 2.94(3H,s), 3.50(3H,s), 5.22(1H,d,J=6.8HZ), 5.29(1H,d,J=6-8Hz), 5.73(1H,brd, J=8.5Hz), 6.01(1H,brd,J=8.6Hz), 6.98(1H,dt,J=7.5Hz, l.lHz), 7.13(lH,m), 7.21-7.33(2H,m)

### Reference Example 7

### α -tert-Butoxycarbonylamino-2-methoxyphenylacetamide

28% Ammonium hydroxide (3.5ml) was added to a solution of methyl α -tert-butoxycarbonylamino-2-methoxymethoxyphenylacetate (250mg) in methanol (4ml), and the mixture was stirred at 50° C for 12 hours. After the reaction, water was added to the reaction mixture, and the mixture was extracted with chloroform. The organic layer was washed with a saturated aqueous sodium hydrogen carbonate solution, dried over magnesium sulfate, and concentrated *in vacuo* to give α -tert-butoxy-carbonylamino-2-methoxymethoxyphenylacetamide (156mg) as a white solid.
NMR(CDCl₃,400MHz)
δ ppm : 1.42(9H,brs), 3.53(3H,s), 5.20-5.35(3H,m), 5.59(1H, brd,J=6.5Hz), 5.97(1H,brs), 6.24(1H,brs), 7.04(1H,dt,J7.5Hz,l.lHz), 7.12(lH,dd,J=8.3Hz,l.OHz), 7.20-7.30 (lH,m), 7.35(lH,dd,J=7.6Hz,1.7Hz)

### Reference Example 8

### N-Benzyl- α -tert-butoxycarbonylamino-2-methoxymethoxyphenylacetamide

Diethyl cyanophosphonate (0.085ml) was added to a solution of α -tert-butoxycarbonylamino-2-methoxymethoxyphenylacetic acid (lOOmg) and benzylamine (0-045ml) in dichloromethane (lOml) under ice-cooling, and the mixture was stirred for 2.5 hours at room temperature. After the reaction, water was added to the reaction mixture, and the mixture was extracted with ethyl acetate. The organic layer was washed successively with a saturated aqueous sodium hydrogen carbonate solution, a 10% aqueous citric acid solution and brine, dried over magnesium sulfate, and concentrated *In vacuo*. The obtained residue was purified by column chromatography on silica gel to give N-benzyl-α -tert-butoxycarbonylamino-2-methoxymethoxyphenylacetamide (99mg) as a white solid.
NMR(CDCl₃,400MHz)
δ ppm : 1.42(9H,brs), 3.30(3H,s), 4.30-4.50(2H,m), 5.10-5.20 (2H,m), 5.61(lH,brd,J=7.7Hz), 6.05(lH,br), 6.59 (lH,br),7.00-7.15(4H,m), 7.20-7.30(4H,m), 7.36(1H,dd, J=7.7Hz,1.7Hz)

### Reference Example 9

### 2-tert-Butoxycarbonylamino-2-(2-methoxymethoxyphenyl)ethanol.

Lithium chloride (400mg) and sodium borohydride (250mg) were added to a solution of methyl α -tert-butoxycarbonylamino-2-methoxymethoxyphenylacetate (1.2g) in tetrahydrofuran (6ml) at room temperature. To the suspension was added ethanol (12ml), and the solution was stirred overnight at room temperature. The reaction mixture was quenched with a saturated aqueous ammonium chloride solution, and the solvent was removed *in vacuo*. Water was added to the residue, and the mixture was extracted with chloroform. The organic layer was washed with brine, dried over magnesium sulfate, and concentrated *in vacuo*. The obtained residue was purified by column chromatography on silica gel to give 2-tert-butoxycarbonylamino-2-(2-methoxymethoxyphenyl)ethanol (1.02g) as a white amorphous powder.
NMR(CDC1₃,400NHz)
δ ppm : 1.42(9H,s), 3.46(3H,s), 3.72-3.80(1H,m), 3.83-3.89 (2H,m), 5.08-5.10(1H,br), 5.22(2H,s), 5.48-5.50(1H, br), 6.98(1H,dt,J=7.2Hz,l.OHz), 7.12(1H,dd,J=8.3Hz, l.lHz), 7.23-7.26(2H,m)

### Reference Example 10

### Methyl 4-tert-butoxycarbonylamino-4-(2-methoxymethoxyphenyl)crotonate

Dimethyl sulfoxide (2ml) and triethylamine (lml) were added to a solution of 2-tert-butoxycarbonylamino-2-(2-methoxymethoxyphenyl)ethanol (l.Og) in dichloromethane (2Oml) at room temperature, and sulfur trioxide pyridine complex (2g) was added slowly to the mixture under ice-cooling. The mixture was stirred for 15 minutes and then for l hour at room temperature. After the reaction mixture was quenched with lN hydrochloric acid, water was added, and the mixture was extracted with dichloromethane. The organic layer was washed with brine, dried over magnesium sulfate, and concentrated *in vacuo* to obtain the corresponding aldehyde. Sodium hydride (60% dispersion in mineral oil) (186mg) was added to a solution of trimethyl phosphonoacetate (0.7ml) in tetrahydrofuran (25ml) under an atmosphere of argon and ice-cooling, and the mixture was stirred for 5 minutes under ice-cooling to prepare a phosphorus reagent. To the reagent was added dropwise the solution of the above aldehyde in tetrahydrofuran (25ml) under ice-cooling. The mixture was stirred at the same temperature for 10 minutes and then for 30 minutes with warming to room temperature. After the reaction mixture was quenched with a saturated aqueous ammonium chloride solution, water was added, and the mixture was extracted with ethyl acetate. The organic layer was washed with brine, dried over magnesium sulfate and concentrated *in vacuo*. The obtained residue was purified by column chromatography on silica gel to give methyl 4-tert-butoxycarbonylamino-4-(2-methoxymethoxyphenyl)crotonate (780mg) as a white solid.
NMR(CDCl₃,400MHz)
δ ppm: 1.41(9H,s), 3.46(3H,s), 3.71(3H,s), 5.20(1H,d,J=6.8Hz), 5.24(lH,d,J=6.8Hz), 5.37(lH,brd,J=7.6Hz), 5.65(lH,brd, J=7.6Hz), 5.93(lH,dd,J=15.8Hz,1.8Hz), 6.98(lH,t, J=7.6Hz), 7.06(1H,d,J=4.8Hz), 7.11(1H,d,J=8.0Hz), 7.18-7.29(2H,m)

### Reference Example 11

### Methyl 4-tert-butoxycarbonylamino-4-(2-methoxymethoxyphenyl)butyrate

Methyl-4-tert-butoxycarbonylamino-4-(2-methoxymethoxyphenyl)crotonate (400mg) was dissolved in methanol (20ml), and the solution was stirred overnight at room temperature in the presence of 10% palladium on activated carbon (lOOmg) under an atmosphere of hydrogen at atmospheric pressure. After the palladium catalyst was filtered off with celite, the filtrate was concentrated in vacuo to give methyl 4-tert-butoxycarbonyl-amino-4-(2-methoxymethoxyphenyl)butyrate (360mg) as a white solid.
NMR(CDCl₃,400MHz)
δ ppm : 1.41(9H,S), 2.04-2.18(2H,m), 2.20-2.38(2H,m), 3.49(3H,s), 3.63(3H,s), 4.89(1H,q,J=7.5Hz), 5.25(2H,s), 5.42(lH,brd,J=6.2Hz), 6.92(lH,dt,J=7.SHz,l.lHz), 7.12(lH,dd,J=6.2Hz,l.lHz), 7.15-7.24(2H,m)

### Example 1

### α -Amino-2-hydroxyphenylacetic acid.hydrochloride

α-tert-Butoxycarbonylamino-2-methoxymethoxyl)phenylacetic acid (250mg) was dissolved in a hydrogen chloride ethanol solution, and the solution was stirred for 1 day at room temperature. After the reaction, the reaction mixture was concentrated *in vacuo*, and the obtained residue was recrystallized from hexane-ethyl acetate-ethanol to give α amino-2-hydroxyphenylacetic acid.hydrochloride (135mg) as a white solid.
NMR(DMSO-d₆,400MHz)
δ ppm : 5.08(1H,s), 6.85(1H,dt,J=6.5Hz,l.OHz), 6.95(1H,d, J=8.1Hz), 7.20-7.32(2H,m), 8.49(3H,brs), 10.4(1H,br), 13.6(lH,br)

### Example 2

### Methyl α -amino-2-hydroxyphenylacetate-hydrochloride

Methyl α -tert-butoxycarbonylamino-2-methoxymethoxyphenylacetate (120mg) was dissolved in a solution (2ml) of hydrogen chloride in methanol, and the solution was stirred for 1 day at room temperature. After the reaction, the reaction mixture was concentrated *in vacuo* to give methyl a-amino-2-hydroxyphenylacetate-hydrochloride (77mg) as a white solid.
NMR(DMSO-d₆,400MHz)
δ ppm : 3.70(3H,s), 5.24(1H,brs), 6.85(1H,dt,J=7.5Hz,l.lHz), 6.97(lH,dd,J=8.lHz,0.9Hz), 7.21-7.35(2H,m), 8.66 (3H,brs), 10.47(1H,s)

### Example 3

### α -Amino-5-chloro-2-hydroxyphenylacetic acid-hydrochloride

In the same manner as that in Reference Examples 1-3 and Example 1, α-amino-5-chloro-2-hydroxyphenylacetic acidhydrochloride was obtained by the use of 5-chlorosalicylaldehyde as a starting material.
a white solid
NMR(DMSO-d₆,400MHz)
δ ppm : 5.08(1H,brs), 6.95(1H,d,J=8.7Hz), 7.29(1H,dd,J=8.7Hz, 2.7Hz), 7.39(lH,d,J=2.7Hz), 8.51(3H,br)

### Example 4

### α -Amino-2-hydroxy-5-methoxyphenylacetic acid-hydrochloride

In the same manner as that in Reference Examples 1-3 and Example 1, α -amino-2-hydroxy-5-methoxyphenylacetic acidhydrochloride was obtained by the use of 2-hydroxy-5-methoxybenzaldehyde as a starting material.
a white solid
NMR(DMSO-d₆,400MHz)
δ ppm : 3.68(3H,s), 5.07(lH,brs), 6.85(2H,s), 6.93(lH,s), 8.49(3H,br), 9.8(1H,br)

### Example 5

### Methyl α -amino-2,5-dihydroxyphenylacetate

α-tert-Butoxycarbonylamino-2,5-dimethoxymethoxyphenylacetic acid (300mg) was dissolved in methanol (lOml), and a 10% hexane solution of trimethylsilyldiazomethane was added dropwise to the solution under ice-cooling. After the reaction, the reaction mixture was concentrated *in vacuo*. To the obtained residue was added a solution (lOml) of hydrogen chloride in isopropanol, and the mixture was stirred for 12 hours at room temperature. The reaction mixture was concentrated *in vacuo*, and the obtained residue was purified by column chromatography on silica gel to give methyl α -amino-2,5-dihydroxyphenylacetate (119mg) as a yellow oil.
NMR(DMSO-d₆,400MHz)
δ ppm : 3.59(3H,s), 4.61(1H,s), 6.49(1H,dd,J=8.6Hz,2.8Hz), 6.51-6.58(2H,m), 8.65(lH,br)

### Example 6

### N,N-Dimethyl- α -amino-2-hydroxyphenylacetamide

Concentrated hydrochloric acid (lml) was added to a solution of N,N-dimethy- α -tert-butoxycarbonylamino-2-methoxymethoxyphenylacetamide (51mg) in chloroform-dioxane (1:1) (lml) under ice-cooling, and the mixture was stirred for 12 hours with raising to room temperature. After the reaction, the reaction mixture was concentrated *in vacuo*, and the obtained residue was purified by column chromatography on silica gel to give N,N-dimethyl- α -amino-2-hydroxyphenyl-acetamide (25mg) as a colorless amorphous powder.
NMR(DMSO-d₆,400MHz)
δ ppm: 2.87(3H,s), 2.88(3H,s), 5.01(1H,s), 6.73(1H,t,J=7.4Hz), 6.78(1H,dd,J=8.0Hz,l.OHz), 6.95(1H,dd,J=7.7Hz,1.6Hz), 7.04-7.10(lH,m)

### Example 7

### α -Amino-2-hydroxyphenylacetamide

Trimethylsilyl bromide (1.66ml) was added to a solution of α-tert-butoxycarbonylamino-2-methoxymethoxyphenylacetamide (880mg) in dichloromethane (14ml), and the mixture was stirred for 1 hour at room temperature. After the reaction, a saturated aqueous sodium hydrogen carbonate solution was added to the reaction mixture, and the mixture was concentrated *in vacuo*. The obtained residue was purified by column chromatography on silica gel to give α -amino-2-hydroxyphenylacetamide (65mg) as a yellow solid.
NMR(DMSo-d₆,400MHz)
δ ppm : 4.53(1H,s), 6.70-6.80(2H,m), 7.00-7.35(4H,m)

### Example 8

### N-Benzyl-α-amino-2-hydroxyphenylacetamide-hydrochloride

N-Benzyl- α -tert-butoxycarbonylamino-2-methoxymethoxy -phenylacetamide (93mg) was dissolved in methanol (4ml), and lN hydrochloric acid (2ml) was added to the solution. The mixture was stirred at 50°C for 2 hours. Concentrated hydrochloric acid (0.05ml) was added to the reaction mixture, and the mixture was stirred for lO minutes. After the reaction, the reaction mixture was concentrated *in vacuo* to give N-benzyl-α -amino-2-hydroxyphenylacetamide.hydrochloride (67mg) as a brown solid.
NMR(DMSO-d₆,400MHz)
δ ppm : 4.20-4.40(2H,m), 5.10(1H,brs), 6.86(dt,1H,J=7.5Hz, l.lHz), 6.98(1H,d,J=7.6Hz), 7.10-7.35(7H,m), 8.42 (3H,brs), 8.60(1H,t,J=6.1Hz), 10.39(1H,brs)

### Example 9 N-(2-Phenethyl)-α-amino-2-hydroxyphenylacetamidehydrochloride

In the same manner as that in Reference Example 8 and Example 8, N-(2-phenethyl)-α-amino-2-hydroxyphenylacetamide. hydrochloride was obtained by the use of 2-phenethylamine as a starting material.
a yellow solid
NMR(DMSO-d₆,400MHz)
δ ppm : 2.69(2H,t,J=7.3Hz), 3.15-3.50(2H,m), 5.00(1H,brs), 6.84(1H,t,J=7.6Hz), 6.90-7.00(lH,m),7.05-7.30 (7H,m), 8.15(1H,brs), 8.38(3H,brs), 10.38(1H,brs)

### Example 10

### 3-Amino-3-(2-hydroxyphenyl)propionic acid

A suspension of coumarin (lg) in 28% ammonium hydroxide was stirred for 6 days at room temperature in a sealed tube. The reaction mixture was concentrated at 120°C. Methanol was added to the residue, and the resulting precipitates were collected by filtration and recrystallized from methanol-water to give 3-amino-3-(2-hydroxyphenyl)propionic acid (72mg) as a white solid.
NMR(DMSO-d₆,400MHz)
δ ppm : 2.30-2.50(2H,m), 4.39-4.47(lH,m), 6.75-6.83(2H,m), 7.07-7.15(1H,m), 7.22(1H,d,J=7.8Hz), 8.3-9.0(3H,br)

### Example 11

### α -Amino-2-hydroxyphenylacetic acid

The pH of an aqueous solution (lOml) of α -amino-2hydroxyphenylacetic acid.hydrochloride (3.00g) was adjusted to pH 7-7.5 with a lN aqueous sodium hydroxide solution. The resulting precipitates were collected by filtration, washed with cold water, and dried to give α -amino-2-hydroxyphenylacetic acid (1.12g) as a white solid.
NMR(DMSO-d₆,400MHz)
δ ppm: 4.59(lH,s),6.75-6.90(2H,m),7.05-7.20(2H,m ),7.50-11.0 (2H,br)

### Example 12

### α -Amino-2-hydroxyphenylacetic acid (optical isomer)

An optical isomer of α-amino-2-hydroxyphenylacetic acid was separated from the corresponding racemate by column chromatography (column, SUMICHIRAL OFFICIAL ACTION-5000 04.6mm x 15cm; mobile phase, 2mM aqueous copper (II) sulfate solution / acetonitrile (85:15); flow rate, 1.O ml / min.; column temperature, 25 C; detector, 254nm). Copper in the fraction having retention time of about 4.5 minutes was removed with SUMICHELATE MC-75, and the pH of the resulting aqueous solution was adjusted to pH 6 approximately. The mixture was concentrated *in vacuo*, and the obtained residue was purified by ODS column chromatography and lyophilized to give α -amino-2-hydroxyphenylacetic acid (optical isomer) as a colorless amorphous powder.
NMR(D₂0,400MHz)
δ ppm : 4.80(lH,s), 6.90-7.05(2H,m), 7.25-7.40(2H,m)

### Example 13

### α -Amino-2-hydroxyphenylacetic acid (optical isomer)

An optical isomer of α-amino-2-hydroxyphenylacetic acid was separated from the corresponding racemate by column chromatography (column, SUMICHIRAL OFFICIAL ACTION-5000 04.6mm x 15cm; mobile phase, 2mN aqueous copper (II) sulfate solution / acetonitrile (85:15); flow rate, 1.0 ml / min.; column temperature, 25 C; detector, 254nm). Copper in the fraction having retention time of about 6.0 minutes was removed with SUMICHELATE MC-75, and the pH of the resulting aqueous solution was adjusted to pH 6 approximately. The mixture was concentrated *in vacuo*, and the obtained residue was purified by ODS column chromatography and lyophilized to give α -amino-2-hydroxyphenylacetic acid (optical isomer) as a colorless amorphous powder.
NMR(D₂0,400MHz)
δ ppm : 4.80(1H,s), 6.90-7.05(2H,m), 7.25-7.40(2H,m)

### Example 14

### α -Amino-2-hydroxyphenylacetamide-hydrochloride

α-tert-Butoxycarbonylamino-2-methoxymethoxyphenylacetamide (lOOmg) was dissolved in a solution (2ml) of hydrogen chloride in methanol, and the solution was stirred for 30 minutes at room temperature. After the reaction, the solvent was removed *in vacuo* to give α -amino-2-hydroxyphenylacetamide.
hydrochloride (61mg) as a white solid.
NMR(DMSO-d₆,400MHz)
δ ppm : 4.97-5.01(lH,m), 6.87(1H,t,J=7.5Hz), 6.96(1H,d, J=8.3Hz), 7.20-7.30(2H,m), 7.44(1H,s), 7.56(1H,s), 8.33(3H,brs), 10.36(lH,brs)

### Example 15

### Isopropyl 4-amino-4-(2-hydroxyphenyl)butyrate.hydrochloride

A solution (2ml) of methyl α -tert-butoxycarbonylamino-2-methoxymethoxyphenylbutyrate (350mg) in methanol was added to an aqueous lithium hydroxide monohydrate (45mg) solution (lml), and the mixture was stirred for 1 hour at room temperature. After the starting material disappeared, the reaction mixture was neutralized with lN hydrochloric acid and extracted with dichloromethane. The organic layer was washed with brine, dried over magnesium sulfate, and concentrated *in vacuo*. The obtained residue was dissolved in ethanol (3ml), and a solution (lml) of hydrogen chloride in 2-propanol was added to the solution. The mixture was stirred overnight at room temperature, and the reaction mixture was concentrated *in vacuo*. The obtained solid was recrystallized from chloroform-n-hexane to give isopropyl 4-amino-4-(2-hydroxyphenyl)butyrate.hydrochloride (3Omg) as a pale yellow solid.
NMR(DMSO-d₆,400MHz)
δ ppm : 1.10-1.20(6H,m), 2.02-2.21(4H,m), 4.40-4.50(1H,br), 4.80-4.86(1H,m), 6.85(1H,t,J=7.5Hz), 6.93(1H,d, J=8.OHz), 7.15-7.25(lH,m), 7.33(lH,d,J=6.9Hz), 8.37(3H,br), 10.13(lH,brs)

### Example 16

### Maillard reaction inhibiting activity test

The test compound (0.2mM or 2mM), lysozyme (lOmg/ml) and fructose (200mM) were dissolved in 0.5M sodium phosphate buffer (pH7.4), and the solution was incubated at 37°C for l week. After incubation, the sample was separated by SDS-PAGE and stained with Coomassie Brilliant Blue R-250. The percentage of formed dimer to total proteins was measured by densitometry. Inhibiting activities of the test compounds were evaluated from comparing the percentage of formed dimer in the presence of the test compound with that in the absence of the test compound.

| Compound | Inhibiting Activity (%) | |
|---|---|---|
| | Concentration (0.2mM) | Concentration (2mM) |
| Example 1 | 21.2 | 86.1 |
| Example 2 | 30.6 | 67.5 |
| Example 3 | 38.8 | 88.4 |
| Example 4 | 30.9 | 87.4 |
| Example 5 | - | 48.3 |
| Example 6 | 13.0 | 82.1 |
| Example 7 | 38.7 | 89.3 |
| Example 8 | 13.9 | 55.3 |
| Example 9 | - | 80.0 |
| Example 10 | 15.0 | - |
| Example 12 | 22.4 | 83.5 |
| Example 13 | 24.0 | 86.3 |
| Example 14 | 21.4 | 87.6 |
| Example 15 | 2.7 | - |
| aminoguanidine | 2.9 | 17.2 |

### Example 17

### Inhibition Assay for AGE formation in vivo.

Diabetes was induced in male SD rats, 6 weeks of age, by intravenous single injection of streptozotocin (STZ) (50mg/kg) into tail. One week after diabetes induction, α -amino-2-hydroxyphenylacetic acid.hydrochloride or aminoguanidine was given to the diabetic rats by daily oral administration at dosage of 50mg/kg or lOOmg/kg. After 4 weeks of administration, blood samples were collected and AGE content in plasma proteins was measured by ELISA using anti-AGE antibody. AGE content in drug-treated rats were estimated compared with the average content of untreated rats (as 100% control).

Administration of test compounds to STZ-induced diabetic rats lowered AGE content in a dose-dependent manner compared with untreated diabetic rats.

| Group | AGE content |
|---|---|
| Treated rat (50 mg/kg) | 81.6% |
| Treated rat (100mg/kg) | 62.4% |
| Aminoguanidine treated rat | 88.8% |
| Untreated rat | 25.4% |

### Example 18

### Acute toxicity test

Male SD rats, 6 weeks of age and weighing between 140g and 165 g, were conditioned to an overnight fast. α -Amino-2 hydroxyphenylacetic acid.hydrochloride suspended in 0.5% of CMC were given to the rats at a dosage of 2000 mg/kg. Control group rats received 0.5% of CMC alone.

Six hours after administration, all rats were allowed free access to food and water. Seven days after administration, blood samples were collected and tested with hematological examinations. Plasma samples were tested with blood biochemical examinations. In the test group, behavior disorder was not observed until 6 hours after administration and all animals did not die until 7 days after administration. Seven days after administration, no significant difference was observed between the test group and the control group in hematological examinations and blood biochemical examinations.

### Example 19

### Toxicity test by long-term administration

Male SD rats, 6 weeks of age, received STZ (50mg/kg) by single intravenous injection into the tail and were made diabetic. One week after STZ injection, α -amino-2-hydroxyphenylacetic acid-hydrochloride suspended in 0.5% of CMC was given to the diabetic rats by daily oral administration at a dosage of lOOmg/kg. Rats in the control group received O.5% of CMC alone. Compared with the control group, significant reduction in weight and significant increase in number of dead animals were not observed until ten weeks after administration.

| Preparation Example 1 | | |
|---|---|---|
| Tablets | Active compound | 100 mg |
| | Corn starch | 50 mg. |
| | Lactose | 79 mg |
| | Hydroxypropylcellulose | 7 mg |
| | Magnesium stearate | 3 mg |
| | | (Total 230 mg) |

| Preparation Example 2 | | |
|---|---|---|
| Fine granules | Active compound | 100 mg |
| | Mannitol | l90 mg |
| | Corn starch | lOO mg |
| | Hydroxypropylcellulose | 10 mg |
| | | (Total 400 mg) |

| Preparation Example 3 | | |
|---|---|---|
| Capsules | Active compound | 1OO mg |
| | Lactose | 18 mg |
| | Crystalline cellulose | 35 mg |
| | Corn starch | 25 mg |
| | Magnesium stearate | 2 mg |
| | | (Total 180 mg) |

## Claims

1. A Maillard reaction inhibitor comprising, as an active ingredient, a 2-hydroxyphenylalkylamine derivative represented by the general formula: 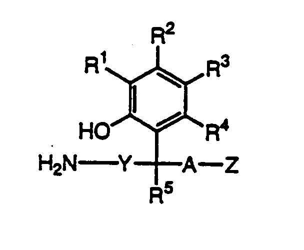 wherein R¹, R², R³ and R⁴ are the same or different and each represents a hydrogen atom, a lower alkyl group, a lower alkoxy group, a hydroxy group, a mercapto group, a halogen atom, a nitro group, an amino group, an acylamino group, an acyl group or a hydroxy(lower alkyl) group; R⁵ represents a hydrogen atom or a lower alkyl group; A represents a single bond, a lower alkylene group which may have a hydroxy group as a substituent or a lower alkenylene group; Y represents a single bond or a lower alkylene group; and Z represents a carboxyl group, a lower alkoxycarbonyl group, an aryloxycarbonyl group, an aralkyloxycarbonyl group, a carbamoyl group, a mono or di(lower alkyl)aminocarbonyl group, a mono or diarylaminocarbonyl group, a mono or diaralkyl aminocarbonyl group, a cyano group, a sulfo group, a lower alkylsulfinyl group, a lower alkylsulfonyl group, an arylsulfinyl group, an arylsulfonyl group, an aralkylsulfinyl group, an aralkylsulfonyl group, a sulfamoyl group, a lower alkyl-sulfamoyl group, an arylsulfamoyl group or an aralkylsulfamoyl group, or a pharmaceutically acceptable salt thereof.

2. A Maillard reaction inhibitor comprising, as an active ingredient, a 2-hydroxyphenylalkylamine derivative as claimed in claim 1, represented by the general formula: 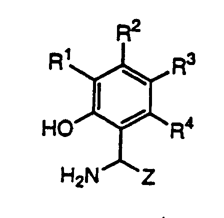 wherein R¹, R², R³ and R⁴ are the same or different and each represents a hydrogen atom, a lower alkyl group, a lower alkoxy group, a hydroxy group, a mercapto group, a halogen atom, a nitro group, an amino group, an acylamino group, an acyl group or a hydroxy(lower alkyl) group; and Z represents a carboxyl group, a lower alkoxycarbonyl group, an aryloxycarbonyl group, an aralkyloxycarbonyl group, a carbamoyl group, a mono or di(lower alkyl)aminocarbonyl group, a mono or diarylaminocarbonyl group, a mono or diaralkylaminocarbonyl group, a cyano group, a sulfo group, a lower alkylsulfinyl group, a lower alkylsulfonyl group, an arylsulfinyl group, arylsulfonyl group, an aralkylsulfinyl group, an aralkylsulfonyl group, a sulfamoyl group, a lower alkylsulfamoyl group, an arylsulfamoyl group or an aralkylsulfamoyl group or a pharmaceutically acceptable salt thereof.

3. A Maillard reaction inhibitor comprising, as an active ingredient, a 2-hydroxyphenylalkylamine derivative as defined in claim 2, represented by the general formula: 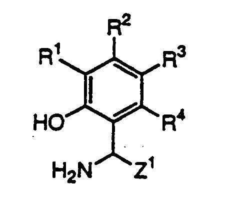 wherein R¹, R², R³ and R⁴ are the same or different and each represents a hydrogen atom, a lower alkyl group, a lower alkoxy group, a hydroxy group, a mercapto group, a halogen atom, a nitro group, an amino group, an acylamino group, an acyl group or a hydroxy(lower alkyl) group; and Z¹ represents a carboxyl group, a lower alkoxycarbonyl group, an aryloxycarbonyl group, an aralkyloxycarbonyl group, a carbamoyl group, a mono or di(lower alkyl)aminocarbonyl group, a mono or diarylaminocarbonyl group, or a mono or diaralkylaminocarbonyl group, or a pharmaceutically acceptable salt thereof.

4. A Maillard reaction inhibitor comprising, as an active ingredient, a 2-hydroxyphenylalkylamine derivative as defined in claim 3, represented by the general formula: 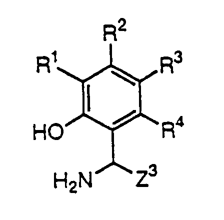 wherein R¹, R², R³ and R⁴ are the same or different and each represents a hydrogen atom, a lower alkyl group, a lower alkoxy group, a hydroxy group, a mercapto group, a halogen atom, a nitro group, an amino group, an acylamino group, an acyl group or a hydroxy(lower alkyl) group; and Z³ represents a carboxyl group, a lower alkoxycarbonyl group, an aryloxycarbonyl group or an aralkyloxycarbonyl group, or a pharmaceutically acceptable salt thereof.

5. A Maillard reaction inhibitor comprising, as an active ingredient, a 2-hydroxyphenylalkylamine derivative as defined in claim 3, represented by the general formula: 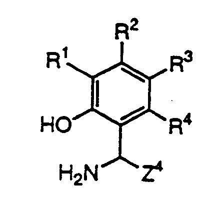 wherein R¹, R², R³ and R⁴ are the same or different and each represents a hydrogen atom, a lower alkyl group, a lower alkoxy group, a hydroxy group, a mercapto group, a halogen atom, a nitro group, an amino group, an acylamino group, an acyl group or a hydroxy(lower alkyl) group; and Z⁴ represents a carbamoyl group, a mono or di(lower alkyl)aminocarbonyl group, a mono or diarylaminocarbonyl group, or a mono or diaralkylaminocarbonyl group or a pharmaceutically acceptable salt thereof.

6. The Maillard reaction inhibitor comprising, as an active ingredient, the 2-hydroxyphenylalkylamine derivative as defined in claim 4, represented by the formula: 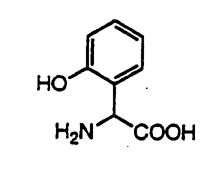 or pharmaceutically acceptable salt thereof.

7. The Maillard reaction inhibitor comprising, as an active ingredient, the 2-hydroxyphenylalkylamine derivative as defined in claim 5, represented by the formula: 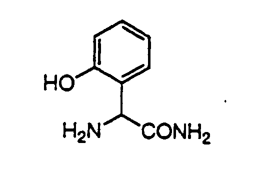 or pharmaceutically acceptable salt thereof.

8. A pharmaceutical composition comprising a 2-hydroxyphenylalkylamine derivative represented by the general formula: 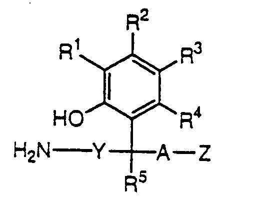 wherein R¹, R², R³ and R⁴ are the same or different and each represents a hydrogen atom, a lower alkyl group, a lower alkoxy group, a hydroxy group, a mercapto group, a halogen atom, a nitro group, an amino group, an acylamino group, an acyl group or a hydroxy(lower alkyl) group; R⁵ represents a hydrogen atom or a lower alkyl group; A represents a single bond, a lower alkylene group which may have a hydroxy group as a substituent or a lower alkenylene group; Y represents a single bond or a lower alkylene group; and Z represents a carboxyl group, a lower alkoxycarbonyl group, an aryloxycarbonyl group, an aralkyloxycarbonyl group, a carbamoyl group, a mono or di(lower alkyl)aminocarbonyl group, a mono or diarylaminocarhonyl group, a mono or diaralkylaminocarbonyl group, a cyano group, a sulfo group, a lower alkylsulfinyl group, a lower alkylsulfonyl group, an arylsulfinyl group, an arylsulfonyl group, an aralkylsulfinyl group, an aralkylsulfonyl group, a sulfamoyl group, a lower alkylsulfamoyl group, an arylsulfamoyl group or an aralkylsulfamoyl group or a pharmaceutically acceptable salt thereof.

9. A pharmaceutical composition as claimed in claim 8, comprising a 2-hydroxy-phenylalkylamine derivative represented by the general formula: 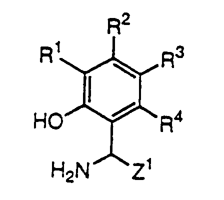 wherein R¹, R², R³ and R⁴ are the same or different and each represents a hydrogen atom, a lower alkyl group, a lower alkoxy group, a hydroxy group, a mercapto group, a halogen atom, a nitro group, an amino group, an acylamino group, an acyl group or a hydroxy(lower alkyl) group; and Z¹ represents a carboxyl group, a lower alkoxycarbonyl group, an aryloxycarbonyl group, an aralkyloxycarbonyl group, a carbamoyl group, a mono or di(lower alkyl)aminocarbonyl group, a mono or diarylaminocarbonyl group, or a mono or diaralkylaminocarbonyl group, or a pharmaceutically acceptable salt thereof.

10. The pharmaceutical composition as claimed in claim 9, comprising the 2-hydroxyphenylalkylamine derivative represented by the formula: 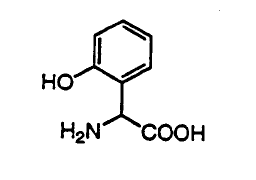 or pharmaceutically acceptable salt thereof.

11. The pharmaceutical composition as claimed in claim 9, comprising the 2-hydroxyphenylalkylamlne derivative represented by the formula: 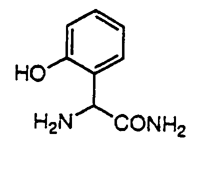 or pharmaceutically acceptable salt thereof.

12. A 2-hydroxyphenylalkylamine derivative represented by the general formula: 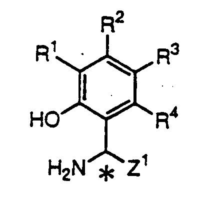 wherein R¹, R², R³ and R⁴ are the same or different and each represents a hydrogen atom, a lower alkyl group, a lower alkoxy group, a hydroxy group, a mercapto group, a halogen atom, a nitro group, an amino group, an acylamino group, an acyl group or a hydroxy(lower alkyl) group; Z¹ represents a carboxyl group, a lower alkoxycarbonyl group, an aryloxycarbonyl group, an aralkyloxycarbonyl group, a carbamoyl group, a mono or di(lower alkyl)aminocarbonyl group, a mono or diarylaminocarbonyl group, or a mono or diaralkylaminocarbonyl group; the carbon atom marked with * represents a carbon atom in (R)-configuration or (S)-configuration; and with the proviso that Z¹ does not represent a carboxy group when one of R² and R³ represents a hydroxy group or methoxy group and the other represents a hydrogen atom and R¹ and R⁴ represent a hydrogen atom, and a pharmaceutically acceptable salt thereof.

13. A 2-hydroxyphenylalkylamine derivative represented by the general formula: 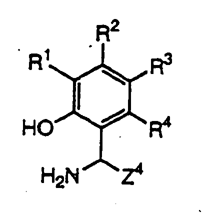 wherein R¹, R², R³ and R⁴ are the same or different. and each represents a hydrogen atom, a lower alkyl group, a lower alkoxy group, a hydroxy group, a mercapto group, a halogen atom, a nitro group, an amino group, an acylamino group, an acyl group or a hydroxy(lower alkyl) group; and Z⁴ represents a carbamoyl group, a mono or di(lower alkyl)aminocarbonyl group, a mono or diarylaminocarbonyl group, or a mono or diaralkylaminocarbonyl group or a pharmaceutically acceptable salt thereof.

14. A method for the prevention or treatment of diseases caused by Maillard reaction which comprise administering a 2-hydroxyphenylalkylamine derivative represented by the general formula: 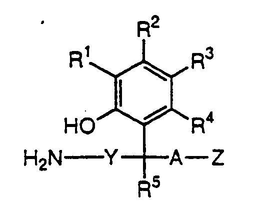 wherein R1 , R², R³ and R⁴ are the same or different and each represents a hydrogen atom, a lower alkyl group, a lower alkoxy group, a hydroxy group, a mercapto group, a halogen atom, a nitro group, an amino group, an acylamino group, an acyl group or a hydroxy(lower alkyl) group; R represents a hydrogen atom or a lower alkyl group; A represents a single bond, a lower alkylene group which may have a hydroxy group as a substituent or a lower alkenylene group; Y represents a single bond or a lower alkylene group; and Z represents a carboxyl group, a lower alkoxycarbonyl group, an aryloxycarbonyl group, an aralkyloxycarbonyl group, a carbamoyl group, a mono or di(lower alkyl)aminocarbonyl group, a mono or diarylaminocarbonyl group, a mono or diaralkylaminocarbonyl group, a cyano group, a sulfo group, a lower alkylsulfinyl group, a lower alkylsulfonyl group, an arylsulfinyl group, an arylsulfonyl group, an aralkylsulfinyl group, an aralkylsulfonyl group, a sulfamoyl group, a lower alkylsulfamoyl group, an arylsulfamoyl group or an aralkylsulfamoyl group or a pharmaceutically acceptable salt thereof.

15. The use of a 2-hydroxyphenylalkylamine derivative represented by the general formula: 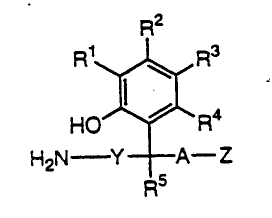 wherein R¹, R², R³ and R⁴ are the same or different and each represents a hydrogen atom, a lower alkyl group, a lower alkoxy group, a hydroxy group, a mercapto group, a halogen atom, a nitro group, an amino group, an acylamino group, an acyl group or a hydroxy(lower alkyl) group; R⁵ represents a hydrogen atom or a lower alkyl group; A represents a single bond, a lower alkylene group which may have a hydroxy group as a substituent or a lower alkenylene group; Y represents a single bond or a lower alkylene group; and Z represents a carboxyl group, a lower alkoxycarbonyl group, an aryloxycarbonyl group, an aralkyloxycarbonyl group, a carbamoyl group, a mono or di(lower alkyl)aminocarbonyl group, a mono or diarylaminocarbonyl group, a mono or diaralkylaminocarbonyl group, a cyano group, a sulfo group, a lower alkylsulfinyl group, a lower alkylsulfonyl group, an arylsulfinyl group, an arylsulfonylgroup, an aralkylsulfonyl group, an aralkylsulfonyl group, a sulfamoyl group, a lower alkylsulfamoyl group, an arylsulfamoyl group or an aralkylsulfamoyl group or pharmaceutically acceptable salt thereof for the manufacture of a pharmaceutical composition for the prevention or treatment of diseases caused by the Maillard reaction.

16. A use of a 2-hydroxyphenylalkylamine derivative represented by the general formula: 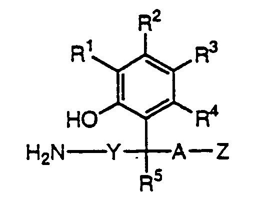 wherein R¹, R², R³ and R⁴ are the same or different and each represents a hydrogen atom, a lower alkyl group, a lower alkoxy group, a hydroxy group, a mercapto group, a halogen atom, a nitro group, an amino group, an acylamino group, an acyl group or a hydroxy(lower alkyl) group; R represents a hydrogen atom or a lower alkyl group; A represents a single bond, a lower alkylene group which may have a hydroxy group as a substituent or a lower alkenylene group; Y represents a single bond or a lower alkylene group; and Z represents a carboxyl group, a lower alkoxycarbonyl group, an aryloxycarbonyl group, an aralkyloxycarbonyl group, a carbamoyl group, a mono or di(lower alkyl)aminocarbonyl group, a mono or diarylaminocarbonyl group, a mono or diaralkylaminocarbonyl group, a cyano group, a sulfo group, a lower alkylsulfinyl group, a lower alkylsulfonyl group, an arylsulfinyl group, an arylsulfonylgroup, an aralkylsulfinyl group, an aralkylsulfonyl group, a sulfamoyl group, a lower alkylsulfamoyl group, an arylsulfamoyl group or an aralkylsulfamoyl group or pharmaceutically acceptable salt thereof as a Maillard reaction inhibitor.
